(19) Europäisches Patentamt
European Patent Office
Office européen des brevets

(11) Veröffentlichungsnummer: **0 180 799**
**B1**

(12) # EUROPÄISCHE PATENTSCHRIFT

(45) Veröffentlichungstag der Patentschrift: **22.06.88**

(51) Int. Cl.[4]: **C 07 C 51/56,** C 07 C 53/12, C 07 C 53/122, B 01 J 23/46

(21) Anmeldenummer: **85112819.9**

(22) Anmeldetag: **10.10.85**

(54) **Verfahren zur Herstellung von Monocarbonsäureanhydriden.**

(30) Priorität: **07.11.84 DE 3440647**

(43) Veröffentlichungstag der Anmeldung: **14.05.86 Patentblatt 86/20**

(45) Bekanntmachung des Hinweises auf die Patenterteilung: **22.06.88 Patentblatt 88/25**

(84) Benannte Vertragsstaaten: **AT BE CH DE FR GB IT LI NL**

(56) Entgegenhaltungen:
**EP - A - 0 075 730**
**FR - A - 2 347 097**
**US - A - 2 722 504**

(73) Patentinhaber: **HOECHST AKTIENGESELLSCHAFT, Postfach 80 03 20, D-6230 Frankfurt am Main 80 (DE)**

(72) Erfinder: **Luft, Gerhard, Dr. Prof., Ludwigstrasse 141a, D-6109 Mühltal (DE)**
Erfinder: **Ritter, Gebhard, Dr., Johann-Peter-Hebelstrasse 11, D-7601 Schutterwald Baden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 180 799 B1

ACTORUM AG

**Beschreibung**

Die Erfindung betrifft ein Verfahren zur Herstellung von Monocarbonsäureanhydriden der allgemeinen Formel $(RCO)_2O$ durch Umsetzung eines Carbonsäureesters oder Dialkylethers der allgemeinen Formeln RCOOR bzw. ROR, wobei R jeweils denselben Alkylrest mit 1–4 C-Atomen bedeutet, mit Kohlenmonoxid in der Gasphase in Gegenwart von Jod oder Brom oder deren Verbindungen als Reaktionspromotor sowie in Gegenwart eines Edelmetallverbindungen aus der Gruppe VIII des Periodensystems enthaltenden Trägerkatalysators bei Temperaturen von 130 bis 400 °C und Drücken von 1–150 bar.

Ein derartiges, in der Gasphase mit einem Trägerkatalysator arbeitendes Verfahren ist bereits aus der DE-Offenlegungsschrift 24 50 965 und der JP-Offenlegungsschrift Nr. 47921/1975 bekannt, welche die bei den Flüssigphase-Verfahren auftretenden Nachteile, z.B. die schwierige Abtrennung und Rückführung von suspendiertem und teilweise gelöstem Katalysator und ggf. Promotor, vermeiden.

In beiden Schriften werden jedoch Gasphase-Verfahren nur mit solchen festen Trägerkatalysatoren beschrieben, welche durch Imprägnierung des Trägermaterials mit Katalysatorlösungen hergestellt wurden. Es ist aber auf diese Weise nicht möglich, z.B. Organostickstoff- oder Organophosphorverbindungen mit dreibindigem Stickstoff bzw. Phosphor im Trägerkatalysator zu fixieren, was sich im allgemeinen auf die Katalysatoraktivität und die Selektivität der Reaktion ungünstig auswirkt.

Vorliegende Erfindung vermeidet diese Mängel durch Anwendung sog. mehrfunktioneller Haftvermittler («Spacer»), in welche Promotoren der V. Hauptgruppe, z.B. Organylamine oder -phosphine, bereits integriert sind. Mit ihrer Hilfe ist es möglich, Edelmetallverbindungen der Gruppe VIII fest an die Trägeroberfläche zu binden (siehe hierzu die bei den nachfolgenden Beschreibungen der Herstellung der Katalysatoren Nr. 1, 9, 10, 11, 13 und 14 genannten Literaturstellen).

Im übrigen ist aus der FR-A-2 347 097 ein Trägerkatalysator mit Aluminiumoxid als Träger von Silicium sowie von Elementen der Gruppen VI A und VIII des Periodensystems, insbesondere von Molybdän oder Wolfram und Cobalt oder Nickel, zur Entschwefelung oder Spaltung von Kohlenwasserstoffen in Gegenwart von Wasserstoff bekannt. Hierbei wird eine organische Halogenverbindung des Siliciums, z.B. Dichlordimethylsilan, vermutlich reaktiv auf die Oberfläche des Trägers aufgebracht, hydrolysiert und bei bevorzugt 500–650 °C gesintert. Anschliessend wird der mit Silicium dotierte Träger mit bevorzugt anorganischen Salzen der übrigen Elemente imprägniert und erneut gesintert, so dass der gesamte Trägerkatalysator aus Metalloxiden besteht.

Im einzelnen ist die Erfindung nun dadurch gekennzeichnet, dass im Trägerkatalysator eine Organosiliciumverbindung mit Alkoxy- oder Halogengruppen sowie mit Organostickstoff-, Organophosphor-, Organoarsen-, Organoschwefel-, Mercapto- oder Thioethergruppen als mehrfunktioneller Haftvermittler einerseits an das Trägermaterial und andererseits an die Edelmetallverbindung gebunden ist.

Darüberinhaus kann das Verfahren der Erfindung wahlweise und bevorzugt dadurch gekennzeichnet sein, dass

a) der Trägerkatalysator zusätzlich Nichtedelmetallverbindungen aus der 1. bis 3. Hauptgruppe oder der 4. bis 6. oder der 8. Nebengruppe des Periodensystems der Elemente als Promotoren enthält;

b) im Trägerkatalysator die Organosiliciumverbindung als mehrfunktioneller Haftvermittler einerseits an das Trägermaterial und andererseits abwechselnd an die Edelmetallverbindung und an eine Nichtedelmetallverbindung aus der 6. oder 8. Nebengruppe des Periodensystems der Elemente gebunden ist;

c) der mehrfunktionelle Haftvermittler eine Organosiliciumverbindung folgender allgemeiner Formeln ist:

I. $R^1_nX_{3-n}Si-(CR^3_2)_m-Y$ oder

II. $R^1_nX_{3-n}Si-(CR^3_2)_m-CHY_2$ oder

III. $[R^1_nX_{3-n}Si-(CR^3_2)_m]_2Z$,

wobei

$X = -Cl$, $-Br$ oder $-OR^2$;

$Y = -NR^4_2$, ein stickstoffhaltiger Arylrest, $-PR^4_2$, $-AsR^4_2$, $-SR^4$ oder $-SH$;

$Z = -NR^4-$, $-PR^4-$, $-AsR^4-$ oder $-S-$;

$R^1 = C_1$ bis $C_5$-Alkyl;

$R^2 = C_1$ bis $C_3$-Alkyl;

$R^3 = H$, $C_1$ bis $C_5$-Alkyl oder $C_6H_5$;

$R^4 = C_1$ bis $C_6$-Alkyl, $C_5$ bis $C_8$-Cycloalkyl oder $-C_6H_5$ oder $C_6H_5CH_2-$, die ggf. mit Halogen-, Methoxy-, Ethoxy- oder $C_1$ bis $C_3$-Alkyl substituiert sind;

$n = 0$ oder 1 oder 2;

$m = 0$ bis 8, vorzugsweise 1 bis 3;

d) der Trägerkatalysator ein anorganisches oxidisches Trägermaterial oder einen Aktivkohleträger enthält, deren restliche aktive Hydroxygruppen durch Veresterung oder Veretherung deaktiviert wurden;

e) der Trägerkatalysator zusammen 0,01 bis 50 Gew%, vorzugsweise 0,1 bis 20 Gew%, Edelmetallverbindung, Haftvermittler und ggf. Nichtedelmetallverbindung enthält;

f) der Trägerkatalysator in einer Korngrösse von 1 bis 20 mm eingesetzt wird.

Als Katalysatorträger kommen bevorzugt anorganische Oxide wie z.B. $SiO_2$, $Al_2O_3$, MgO, $TiO_2$, $La_2O_3$, $ZrO_2$, Zeolith, Ton, NiO, $Cr_2O_3$, $WO_3$ oder entsprechende Mischoxide, aber auch Aktivkohle infrage, welche BET-Oberflächen von 1–1000 $m^2/g$, vorzugsweise 30–400 $m^2/g$, haben und stets noch OH-Gruppen aufweisen. Diese OH-Gruppen reagieren mit der oder den funktionellen Gruppen X des Haftvermittlers unter Bildung von Sauerstoffbrücken zwischen Träger und Haftvermittler. Die Promotoren der 5. oder 6. Hauptgruppe sind im Haftvermittler chemisch gebunden und bilden selbst eine seiner funktionellen Gruppen, an welche die Edelmetallverbindungen aus der Gruppe

VIII, insbesondere Rh, Ir, Pd oder Ru, ggf. abwechselnd mit Nichtedelmetallverbindungen aus der 6. oder 8. Nebengruppe, insbesondere von Cr oder Ni, aber auch von W, Fe und Co, angelagert werden. Hierbei können diese Edelmetall- und ggf. Nichtedelmetallverbindungen Brücken zwischen den einzelnen, am Träger fixierten Haftvermittler-Molekülen bilden.

Es ist ein Vorteil des Verfahrens der Erfindung, dass die zur Steigerung der Katalysatoraktivität und der Selektivität notwendigen Promotoren aus der Hauptgruppe V oder VI des Periodensystems der Elemente eine funktionelle Gruppe Y oder Z in mehrfunktionellen Haftvermittlern bilden und somit bis zur maximalen Konzentration, die durch die Anzahl der OH-Gruppen auf der Trägeroberfläche bestimmt ist, fixiert werden können. Deshalb entfällt eine Abtrennung und Rückführung dieser z.B. Organostickstoff- oder Organophosphorpromotoren. Das Verfahren der Erfindung zur Herstellung von Monocarbonsäureanhydriden weist gegenüber den eingangs beschriebenen bekannten Verfahren, welche bereits in der Gasphase mit einem Trägerkatalysator arbeiten, höhere Katalysatoraktivitäten und Selektivitäten auf.

Das Verfahren der Erfindung dient insbesondere zur Herstellung von Essigsäureanhydrid aus Methylacetat oder Dimethylether in Gegenwart von Methyljodid oder Methylbromid als Reaktionspromotor. Als Reaktionspromotor kann auch HI, HBr oder allgemein RI oder RBr eingesetzt werden, wobei R einen Alkylrest mit 1–4 C-Atomen bedeutet.

In den allgemeinen Formeln für die Organosiliciumverbindungen, welche als Haftvermittler (Spacer) infrage kommen, bedeutet X vorzugsweise $-OR^2$ und insbesondere Methoxy und Ethoxy. Sofern n nicht ohnehin null ist, bedeutet $R^1$ bevorzugt einen unverzweigten Alkylrest, insbesondere Methyl, Ethyl oder Propyl.

Die Trägermaterialien wurden bereits genannt: als Mischoxide kommen z.B. $Cr_2O_3 - Al_2O_3$, $WO_3 - Al_2O_3$, $MgO - Al_2O_3$, $SiO_2 - Al_2O_3$ oder $ZrO_2 - Al_2O_3$ infrage. Der Trägerkatalysator enthält bevorzugt 0,01 bis 5 Gew% Edelmetall.

Als Edelmetallverbindungen kommen bei der Herstellung des Trägerkatalysators z.B. folgende Verbindungen infrage:

Rhodium:
$RhCl_3$, $RhCl_3 \cdot 3\,H_2O$, $RhBr_3$, $RhI_3$, $Rh(NO_3)_3$, $Rh_2(CO)_4Cl_2$, $Rh_2(CO)_4Br_2$, $Rh(CO)_4I_2$, $[P(C_6H_5)_3]_3RhCl$, $[P(C_6H_5)_3]_2$, $Rh(CO)Cl$, $Rh_6(CO)_{16}$, $Rh_4(CO)_{12}$, $Rh_2(O_2CCH_3)_4$, $[RhCl(C_8H_{12})]_2$;

Iridium:
$IrCl_2$, $[Ir(CO)_3Cl]_2$, $Ir\ [P(C_6H_5)_3]_2(CO)Cl$, $Ir_4(CO)_{12}$, $[IrCl(C_8H_{12})]_2$, $Cl(CO)_2Irpyr$ (pyr = $C_6H_5N$);

Palladium:
$PdCl_2$, $PdBr_2$, $PdI_2$, $(CH_3CO_2)_2Pd[P(C_6H_5)_3]_2$, $PdCl_2[P(C_6H_5)_3]_2$, $Pd(O_2CCH_3)_2$, $PdCl_2(C_8H_{12})$, $(C_6H_5CN)_2PdCl_2$;

Ruthenium:
$RuCl_3$, $Ru_3(CO)_{12}$, $RuCl_2[P(C_6H_5)_3]_3$, $RuCl_2(CO)_2[P(C_6H_5)_3]_2$, $[RuCl_2(CO)_3]_2$.

Als Nichtedelmetallverbindungen aus der 6. oder 8. Nebengruppe, insbesondere Cr, Ni, aber auch W, Fe, Co, die ebenfalls an die mehrfunktionellen Haftvermittler angelagert werden können, kommen z.B. ferner infrage:

Chrom:
$Cr(CO)_6$, $CrCl_3$, $C_7H_8Cr(CO)_3$.

Nickel:
$Ni(CO)_4$, $[P(C_6H_5)_3]_2Ni(CO)_2$, $NiCl_2$, $Ni(C_8H_{12})_2$.

Als Nichtedelmetallverbindungen aus der 1. bis 3. Hauptgruppe oder der 4. bis 6. oder der 8. Nebengruppe des Periodensystems, vorzugsweise des Li, Na, Mg, Ca, Al, Ti, Zr, V, Cr, W, Fe, Co, Ni, können z.B. Hydroxide, Carbonate, Carbonyle, Hydride, Halogenide und andere Salze eingesetzt werden. Diese Verbindungen unedler Metalle, z.B. Natriumjodid, können z.B. als Lösung durch Imprägnierung auf den Katalysatorträger aufgebracht werden.

Zur Herstellung des erfindungsgemäss eingesetzten Trägerkatalysators muss zuerst der mehrfunktionelle Haftvermittler (Organosiliciumverbindung) bereitgestellt werden. Dieser ist entweder im Handel erhältlich oder kann nach oder in Analogie zu Literaturangaben dargestellt werden. Im allgemeinen wird jetzt an diese Haftvermittler, und zwar an die Promotorgruppe Y oder Z, welche ein Element aus der 5. oder 6. Hauptgruppe enthält, in an sich bekannter Weise eine der genannten Edelmetallverbindungen aus der Gruppe VIII und gegebenenfalls eine der genannten Nichtedelmetallverbindungen aus der 6. oder 8. Nebengruppe angelagert. Anschliessend erfolgt die reaktive Anlagerung des edelmetallhaltigen Zwischenprodukts an die Hydroxygruppen des Trägermaterials unter Austritt einer Gruppe X als Verbindung XH (z.B. HCl, HBr oder $R^2OH$). Dies wird durch 24- bis 100-stündiges Erhitzen am Rückfluss der in einem unpolaren Lösemittel (z.B. Benzol, Toluol, Xylol) suspendierten Komponenten bis zur Entfärbung erreicht.

Andererseits kann man auch zuerst den mehrfunktionellen Haftvermittler (Organosiliciumverbindung) reaktiv unter Austritt einer Gruppe X als Verbindung XH an die Hydroxygruppen des Trägermaterials anlagern und erst anschliessend die Anlagerung der Edelmetallverbindung aus der Gruppe VIII und ggf. einer der genannten Nichtedelmetallverbindungen aus der 6. oder 8. Nebengruppe an die Promotorgruppe Y oder Z des Zwischenprodukts vornehmen.

Alle Einzelheiten ergeben sich aus der Katalysatorbeschreibung. Besonders bei diskontinuierlichem und in der Anfahrphase bei kontinuierlichem Betrieb ist es zwecks Erhöhung der Selektivität und Unterdrückung von Nebenreaktionen sinnvoll, die restlichen OH-Gruppen auf der Oberfläche des Katalysatorträgers, welche nicht mit

den funktionellen Gruppen X des Haftvermittlers reagiert haben, zu desaktivieren. Dies kann z.B. durch Silylierung mit Trimethylchlorsilan, Methylierung mit Methyljodid oder Acetylierung mit Essigsäureanhydrid geschehen.

Das Mengenverhältnis von Carbonsäureester bzw. Dialkylether und Jod(verbindung) oder Brom(verbindung) in der Reaktionszone kann innerhalb weiter Grenzen schwanken. Im allgemeinen beträgt die Menge des Carbonsäureesters und/oder Dialkylethers 1 bis 500 Mol, vorzugsweise 1 bis 100 Mol, je 1 Mol Jod(verbindung) oder Brom(verbindung). Die Temperatur der Reaktionszone wird so gewählt, dass das Reaktionsgemisch bei jedem beliebigen Umsatz gasförmig vorliegt. Bevorzugt wird die Temperatur zwischen 170 und 250 °C gewählt. Der bevorzugte Druck liegt zwischen 10 und 40 bar.

Die Verweilzeit des Reaktionsgemisches am festen Trägerkatalysator beträgt 1 bis 1000 s, bevorzugt 1 bis 180 s. Die Umsetzung kann in einem vorzugsweise senkrecht angeordneten und mit Trägerkatalysator gefüllten Strömungsrohr oder auch in einem Rühr- oder Schüttelautoklaven erfolgen, der den Trägerkatalysator enthält. Man führt die Carbonylierung im allgemeinen unter praktisch wasserfreien Bedingungen durch, doch ist die Anwesenheit geringer Wassermengen, wie sie in den handelsüblichen Ausgangsstoffen vorkommen, zulässig, sollte aber 1 Mol%, berechnet auf die Ausgangsstoffe, nicht übersteigen. Auch wird die Carbonylierung durch geringe Mengen Methanol in den Ausgangsstoffen nicht beeinträchtigt. Ebensowenig stört Wasserstoff, der in kleinen Mengen im handelsüblichen Kohlenmonoxid vorhanden sein kann.

Das aus der Carbonylierungszone abströmende Reaktionsgemisch ist gasförmig und enthält Kohlenmonoxid, Methyljodid, Essigsäureanhydrid, nicht-umgesetztes Methylacetat oder Dimethylether und ggf. geringe Mengen Essigsäure. Das gasförmige Reaktionsgemisch wird abgekühlt, wobei Essigsäureanhydrid und ggf. Essigsäure auskondensieren. Die nicht kondensierten Gase wie CO, $CH_3I$, Methylacetat oder Dimethylether werden in die Reaktionszone zurückgeführt, wobei die umgesetzten Anteile von Ester oder Ether sowie CO fortlaufend ersetzt werden. Die einfache Abtrennung der Anhydride durch Kühlung des abströmenden Reaktionsgemisches und Rückführung der nicht kondensierbaren Gase stellt einen wesentlichen Vorteil des erfindungsgemässen Verfahrens dar, da dies ohne komplizierte Trennoperationen erfolgen kann. Der Trägerkatalysator wird nicht verunreinigt und verbleibt in der Reaktionszone, was den gesamten Verfahrensablauf bedeutend vereinfacht.

Beispiele

Druckautoklavversuche:

Man verwendet einen 0,25 Liter fassenden Rührautoklav aus korrosionsfreiem Edelstahl (Hastelloy C) mit den erforderlichen Zu- und Ableitungen, der einen drehbaren Katalysatorkorb enthält. Die Carbonsäureester oder Dialkylether werden gasförmig in Gegenwart des bewegten, festen Trägerkatalysators mit CO-Gas umgesetzt. Der Trägerkatalysator befindet sich in dem drehbaren Katalysatorkorb, der gleichzeitig für die Durchmischung der Gase sorgt. Der Autoklav wird mit 2,5 ml eines flüssigen Gemisches aus 20 Volumenteilen Methyljodid und 80 Volumenteilen Ester oder Ether beschickt und auf Reaktionstemperatur aufgeheizt. Die Carbonylierung wird durch Aufpressen von Kohlenmonoxid eingeleitet. Der CO-Druck wird durch regelmässiges Nachdrücken konstant gehalten. Die Einzelheiten ergeben sich aus den einzelnen Beispielen.

Beispiel 1

2 ml (1,86 g) Essigsäuremethylester, 0,5 ml (1,14 g) Methyljodid und 1,69 g Katalysator Nr. 1 werden im Autoklaven mit Kohlenmonoxid bei 20 bar CO-Druck und 200 °C umgesetzt. Nach 1 h Reaktionszeit ergibt sich eine Raum-Zeit-Ausbeute von 4 g $Ac_2O/R_{Rh} \cdot h$. Die Ausbeute von $Ac_2O$, bezogen auf den eingesetzten Ester, beträgt 4% bei einer Selektivität von 90%.

Beispiel 2

2 ml (1,86 g) Essigsäuremethylester, 0,5 ml (1,14 g) Methyljodid und 1,58 g Katalysator Nr. 2 werden im Autoklaven mit Kohlenmonoxid bei 20 bar CO-Druck und 100 °C umgesetzt. Nach 1 h Reaktionszeit ergibt sich eine Raum-Zeit-Ausbeute von 80 g $Ac_2O/g_{Rh} \cdot h$. Die Ausbeute von $Ac_2O$, bezogen auf den eingesetzten Ester, beträgt 34,6% bei einer Selektivität von 96%.

Beispiel 3

2 ml (1,86 g) Essigsäuremethylester, 0,5 ml (1,14 g) Methyljodid und 1,92 g Katalysator Nr. 3 werden im Autoklaven mit Kohlenmonoxid bei 20 bar CO-Druck und 200 °C umgesetzt. Nach 1 h Reaktionszeit ergibt sich eine Raum-Zeit-Ausbeute von 30 g $Ac_2O/g_{Rh} \cdot h$. Die Ausbeute von $Ac_2O$, bezogen auf den eingesetzten Ester, beträgt 18% bei einer Selektivität von 81%.

Beispiel 4

2 ml (1,86 g) Essigsäuremethylester, 0,5 ml (1,14 g) Methyljodid und 1,95 g Katalysator Nr. 4 mit einem $Al_2O_3/Cr_2O_3$-Träger (Gewichtsverhältnis 5:1) werden im Autoklaven mit Kohlenmonoxid bei 20 bar CO-Druck und 200 °C umgesetzt. Nach 1 h Reaktionszeit ergibt sich eine Raum-Zeit-Ausbeute von 140 g $Ac_2O/g_{Rh} \cdot h$. Die Ausbeute von $Ac_2O$, bezogen auf den eingesetzten Ester, beträgt 42,8% bei einer Selektivität von 97.5%.

Beispiel 5

2 ml (1,86 g) Essigsäuremethylester, 0,5 ml (1,14 g) Methyljodid und 1,9 g Katalysator Nr. 5 mit einem $Al_2O_3/WO_3$-Träger (Gewichtsverhältnis 10:1) werden im Autoklaven mit Kohlenmonoxid bei 20 bar CO-Druck und 200 °C umgesetzt. Nach 1 h Reaktionszeit ergibt sich eine Raum-Zeit-Ausbeute von 90 g $Ac_2O/g_{Rh} \cdot h$. Die Ausbeute von $Ac_2O$, bezogen auf den eingesetzten Ester, beträgt 44,2% bei einer Selektivität von 94%.

Beispiel 6

2 ml (1,86 g) Essigsäuremethylester, 0,5 ml (1,14 g) Methyljodid und 1,66 g Katalysator Nr. 6, dessen Aluminiumoxid-Träger mit einer wässrigen NaI-Lösung imprägniert und anschliessend getrocknet worden war (Gewichtsverhältnis $Al_2O_3$:NaI = 30:1), werden im Autoklaven mit Kohlenmonoxid bei 20 bar CO-Druck und 200 °C umgesetzt. Nach 1 h Reaktionszeit ergibt sich eine Raum-Zeit-Ausbeute von 130 g $Ac_2O/g_{Rh} \cdot h$. Die Ausbeute von $Ac_2O$, bezogen auf den eingesetzten Ester, beträgt 20,3% bei einer Selektivität von 99%.

Beispiel 7

2 ml (1,86 g) Essigsäuremethylester, 0,5 ml (1,14 g) Methyljodid und 2,65 g Katalysator Nr. 7 werden im Autoklaven mit Kohlenmonoxid bei 20 bar CO-Druck und 200 °C umgesetzt. Nach 1 h Reaktionszeit ergibt sich eine Raum-Zeit-Ausbeute von 70 g $Ac_2O/g_{Rh} \cdot h$. Die Ausbeute von $Ac_2O$, bezogen auf den eingesetzten Ester, beträgt 14,5% bei einer Selektivität von 88%.

Beispiel 8

Ein Stahlrohr von 20 mm Durchmesser und 450 mm Länge wird als Strömungsrohr senkrecht angeordnet und mit 35 g Katalysator Nr. 8 gefüllt. Bei einem Druck von 10 bar und 180 °C werden stündlich 30 Nl CO(Nl = Liter, gemessen bei 1,013 bar und 0 °C) sowie ein verdampftes Gemisch (10 ml Flüssigkeit) aus Methylacetat und Methyljodid (Molverhältnis 11:1) durch das Strömungsrohr geleitet.

Das abströmende Reaktionsgemisch wird unter Normaldruck auf 0 °C abgekühlt und gaschromatographisch analysiert. Hierbei ergibt sich eine Raum-Zeit-Ausbeute von 11,2 g $Ac_2O/g_{Rh} \cdot h$. Die Ausbeute von $Ac_2O$, bezogen auf den eingesetzten Ester, beträgt 18,7% bei einer Selektivität von 98%.

Unter diesen Reaktionsbedingungen wurde die Carboxylierung 200 Stunden durchgeführt, wobei der eingesetzte Trägerkatalysator keinen Aktivitätsverlust zeigte.

Beispiel 9

2 ml (1,86 g) Essigsäuremethylester, 0,5 ml (1,14 g) Methyljodid und 1,93 g Katalysator Nr. 9 werden im Autoklaven mit Kohlenmonoxid bei 20 bar CO-Druck und 200 °C umgesetzt. Nach 1 h Reaktionszeit ergibt sich eine Raum-Zeit-Ausbeute von 20 g $Ac_2O/g_{Pd} \cdot h$. Die Ausbeute von $Ac_2O$, bezogen auf den eingesetzten Ester, beträgt 5,6% bei einer Selektivität von 99,5%.

Beispiel 10

2 ml (1,86 g) Essigsäuremethylester, 0,5 ml (1,14 g) Methyljodid und 1,92 g Katalysator Nr. 10 werden im Autoklaven mit Kohlenmonoxid bei 20 bar CO-Druck und 200 °C umgesetzt. Nach 1 h Reaktionszeit ergibt sich eine Raum-Zeit-Ausbeute von 10 g $Ac_2O/g_{Ru} \cdot h$. Die Ausbeute von $Ac_2O$, bezogen auf den eingesetzten Ester, beträgt 3,61% bei einer Selektivität von 99,5%.

Beispiel 11

2 ml (1,86 g) Essigsäuremethylester, 0,5 ml (1,14 g) Methyljodid und 3,5 g Katalysator Nr. 11 werden im Autoklaven mit Kohlenmonoxid bei 20 bar CO-Druck und 200 °C umgesetzt. Nach 1 h Reaktionszeit ergibt sich eine Raum-Zeit-Ausbeute von 20 g $Ac_2O/g_{Ir} \cdot h$. Die Ausbeute von $Ac_2O$, bezogen auf den eingesetzten Ester, beträgt 11,4% bei einer Selektivität von 97,5%.

Beispiel 12

1,86 g Dimethylether, 0,5 ml (1,14 g) Methyljodid und 1,92 g Katalysator Nr. 12 werden im Autoklaven mit Kohlenmonoxid bei 20 bar CO-Druck und 200 °C umgesetzt. Die Raum-Zeit-Ausbeute beträgt 30 g $Ac_2O/g_{Rh} \cdot h$. Die Ausbeute von $Ac_2O$ nach 2 h, bezogen auf den eingesetzten Ether, beträgt 12,7% bei einer Selektivität von 66%.

Das Hauptnebenprodukt ist Essigsäuremethylester.

Beispiel 13

2 ml (1,86 g) Essigsäuremethylester, 0,5 ml (1,14 g) Methyljodid und 1,6 g Katalysator Nr. 13 werden im Autoklaven mit Kohlenmonoxid bei 20 bar CO-Druck und 200 °C umgesetzt. Nach 1 h Reaktionsdauer ergibt sich eine Raum-Zeit-Ausbeute von 110 g $Ac_2O/g_{Rh} \cdot h$. Die Ausbeute von $Ac_2O$, bezogen auf den eingesetzten Ester, beträgt 38% bei einer Selektivität von 93%.

Beispiel 14

2 ml (1,86 g) Essigsäuremethylester, 0,5 ml (1,14 g) Methyljodid und 1,6 g Katalysator Nr. 14 werden im Autoklaven mit Kohlenmonoxid bei 20 bar CO-Druck und 200 °C umgesetzt. Nach 1 h Reaktionsdauer ergibt sich eine Raum-Zeit-Ausbeute von 120 g $Ac_2O/g_{Rh} \cdot h$. Die Ausbeute von $Ac_2O$, bezogen auf den eingesetzten Ester, beträgt 38% bei einer Selektivität von 96%.

Beschreibung der Katalysatorherstellung

In allen Fällen wurden die Katalysatorträger zwecks Aktivierung zuvor bei 200 °C und etwa 0,133 mbar 10 h getrocknet. Alle Synthesen wurden in Gegenwart von Stickstoff unter Ausschluss von Sauerstoff und Wasser durchgeführt, wobei sämtliche Reagenzien zuvor über Molekularsieb 4 A getrocknet worden waren.

Zur Unterdrückung von Nebenreaktionen und Verbesserung der Selektivität wurden die nachfolgend beschriebenen Katalysatoren abschliessend mit Trimethylchlorsilan behandelt.

$$T\text{–}OH + Cl\text{–}Si(CH_3)_3 \xrightarrow[-HCl]{58\,°C} T\text{–}O\text{–}Si(CH_3)_3$$

Zu diesem Zweck wurden alle hergestellten Katalysatoren bei Raumtemperatur mit Trimethylchlorsilan vollständig bedeckt. Diese Suspension wurde zum Sieden erhitzt und so lange unter Rückfluss gekocht bis keine Gasentwicklung mehr auftrat. Anschliessend liess man die Suspension abkühlen, trennte den Katalysator von der Flüssigkeit ab und trocknete ihn bei 85 °C und 1,33 mbar 12 Stunden lang.

Das in den nachfolgenden Formeln gebrauchte Symbol «Ø» steht für den Phenylrest ($C_6H_5$).

Katalysator Nr. 1

$$[SiO_2\text{-})\text{-}O\text{-}\underset{OC_2H_5}{\overset{OC_2H_5}{Si}}\text{-}CH_2CH_2\text{-}PØ_2]_2RhCOCl$$

20 g aktiviertes Siliciumdioxid mit einem Durchmesser von 3 mm, einer inneren Oberfläche nach BET von 300 $m^2/g$ und einem Porenvolumen von 0,95 ml/g wurden mit 30 ml Benzol versetzt. Zu dieser Suspension tropfte man 3,35 g (37,5 mg Rh) der Verbindung $[(C_2H_5O)_3SiCH_2CH_2PØ_2]_2RhCOCl$ (hergestellt aus $(C_2H_5O)_3SiCH_2CH_2PØ_2$ und $[Rh(CO)_2Cl]_2$, vgl. K.G. Allum, J. Organometallic Chem. 87 (1975) S. 203–216; zur Herstellung von $(C_2H_5O)_3SiCH_2CH_2PØ_2$ aus Triethoxyvinylsilan und Diphenylphosphin unter UV-Belichtung siehe H. Niebergall, Makromol. Chem. 52 (1962) S. 218; zur Herstellung von $[Rh(CO)_2Cl]_2$ aus $RhCl_3 \cdot 3\ H_2O$ und CO-Gas siehe J. A. Mc. Cleverty et al, Inorg. Synth. 8 (1966), S. 211), die in Benzol gelöst ist, unter Rühren hinzu und erhitzte die Mischung bis zum Sieden. Die gelbe Lösung war nach 24 Stunden Rückfluss vollständig entfärbt. Das Lösemittel Benzol wurde abgesaugt und der gelbliche Katalysator in die Soxhlet-Apparatur überführt. Nach 12 h Soxhlet-Extraktion mit Benzol als Extraktionsmittel wurde der Katalysator bei 1,33 mbar und 85 °C getrocknet und anschliessend mit Trimethylchlorsilan weiterbehandelt. Die eingeengten Lösungen enthielten kein Rhodium mehr. Der Rhodiumgehalt des so hergestellten Katalysators beträgt 1,5 Gew%.

Katalysator Nr. 2

$$[Al_2O_3\text{-})\text{-}O\text{-}\underset{OC_2H_5}{\overset{OC_2H_5}{Si}}\text{-}CH_2CH_2PØ_2]_2RhCOCl$$

3 g aktivierte Aluminiumoxidkugeln mit einem Durchmesser von 3 mm, einer inneren Oberfläche nach BET von 125 $m^2/g$ und einem Porenvolumen von 0,9 ml/g wurden zu 200 mg (22,4 mg Rh) der Verbindung $[(C_2H_5O)_3SiCH_2CH_2PØ_2]_2RhCOCl$, gelöst in 20 ml Xylol, unter Rühren hinzugegeben. Die Suspension wurde bis zum Sieden erhitzt. Nach 48 h Rückfluss war die gelbe Lösung vollständig entfärbt. Das Lösemittel Xylol wurde abgesaugt und der gelbliche Katalysator in die Soxhlet-Apparatur überführt. Nach 12 h Soxhlet-Extraktion mit Benzol wurde der Katalysator bei 1,33 mbar und 85 °C acht Stunden getrocknet und anschliessend mit Trimethylchlorsilan weiterbehandelt. Die eingeengten Lösungen enthielten kein Rhodium mehr. Der Rhodiumgehalt des so hergestellten Katalysators beträgt 0,7 Gew%.

Katalysator Nr. 3

$$[Al_2O_3\text{-})\text{-}O\text{-}\underset{OCH_3}{\overset{OCH_3}{Si}}\text{-}CH_2CH_2CH_2S]_2Rh_2(CO)_4$$

Zu 3,2 g getrockneten Aluminiumoxidkugeln mit einem Durchmesser von 3 mm, einer inneren Oberfläche nach BET von 125 $m^2/g$ und einem Porenvolumen von 0,9 ml/g gab man 3 ml (0,016 mol) der im Handel erhältlichen Verbindung $(CH_3O)_3SiCH_2CH_2CH_2SH$, welche in 20 ml Toluol gelöst war, hinzu und erhitzte zum Sieden. Nach 24 h Rückfluss wurde die Lösung unter vermindertem Druck abdestilliert und der Rückstand in die Soxhlet-Apparatur überführt. Nach 12 h Soxhlet-Extraktion mit Benzol wurden die Pellets bei 85 °C und 1 Torr über Nacht getrocknet. Zu 3,1 g dieser Pellets gab man bei Raumtemperatur 50 mg der Verbindung $Rh_2(CO)_4Cl_2$ in Benzol, wobei sofort nach Zugabe Chlorwasserstoff entwich. Nach 20 h war die überstehende benzolische Lösung vollständig entfärbt, wobei die Pellets die rötliche Farbe der Lösung angenommen hatten. Die rötlich-braunen Pellets wurden abfiltriert und in die Soxhlet-Apparatur überführt, 12 h mit Benzol extrahiert, bei 1,33 mbar und 85 °C getrocknet und anschliessend mit Trimethylchlorsilan weiterbehandelt. Der Rh-Gehalt des Katalysators beträgt 0,8 Gew%.

Katalysator Nr. 4

$$[{}^{Al_2O_3}_{Cr_2O_3}\text{-})\text{-}O\text{-}\underset{OC_2H_5}{\overset{OC_2H_5}{Si}}CH_2CH_2PØ_2]_2RhCOCl$$

Zu einer Mischung aus 7 g aktiviertem Aluminiumoxid und 19 Gew% Chrom(III)oxid mit einer inneren Oberfläche nach BET von 60 $m^2/g$ und einem Korndurchmesser von 2 mm gab man 285 mg (31,9 mg Rh) der Verbindung $[(C_2H_5O)_3SiCH_2CH_2PØ_2]_2RhCOCl$, gelöst in 20 ml Xylol, unter Rühren hinzu. Diese Suspension wurde zum Sieden erhitzt. Nach 48 h Rückfluss war die gelbe Lösung vollständig entfärbt. Das Lösemittel Xylol wurde unter vermindertem Druck abdestilliert und der grünliche Katalysator in die Soxhlet-Apparatur überführt. Nach 12 h Soxhlet-Extraktion mit Benzol wurde der Katalysator bei 1,33 mbar und 85 °C 8 Stunden getrocknet und anschliessend mit Trimethylchlorsilan weiterbehandelt. Die eingeengten Lösemittel enthielten kein Rhodium mehr. Der Rhodiumgehalt des so hergestellten Katalysators beträgt 0,4 Gew%.

Katalysator Nr. 5

$$[{}^{Al_2O_3}_{WO_3}\text{-})\text{-}O\text{-}\underset{OC_2H_5}{\overset{OC_2H_5}{Si}}CH_2CH_2PØ_2]_2RhCOCl$$

Zu einer Mischung aus 2 g aktiviertem Aluminiumoxid und 10 Gew% Wolframoxid mit einer inneren Oberfläche nach BET von 140 $m^2/g$ und

einem Korndurchmesser von 2 mm gab man 125 mg (14 mg Rh) der Verbindung $[(C_2H_5O)_3SiCH_2CH_2P\text{Ø}_2]_2RhCOCl$, gelöst in 20 ml Xylol, unter Rühren hinzu. Die Suspension wurde bis zum Sieden erhitzt. Nach 48 h Rückfluss war die gelbe Lösung vollständig entfärbt. Das Lösemittel Xylol wurde unter vermindertem Druck abdestilliert und der Katalysator in die Soxhlet-Apparatur überführt. Nach 12 h Soxhlet-Extraktion mit Benzol wurde der Katalysator bei 1,33 mbar und 85 °C acht Stunden getrocknet und anschliessend mit Trimethylchlorsilan weiterbehandelt. Der Rhodiumgehalt des so hergestellten Katalysators beträgt 0,66 Gew%.

Katalysator Nr. 6

$$[Al_2O_3\text{-})\text{-}O\text{-}\overset{OC_2H_5}{\underset{OC_2H_5}{Si}}\text{-}CH_2CH_2\text{-}P\text{Ø}_2]_2RhCOCl \; + NaI$$

Zu 3,1 g aktiviertem Aluminiumoxid (99% $Al_2O_3$) mit einem Korndurchmesser von 3 mm, einer inneren Oberfläche nach BET von 125 $m^2/g$ und einem Porenvolumen von 0,9 ml/g gab man 0,1 g Natriumjodid, gelöst in 30 ml Aceton, unter Rühren hinzu und erhitzte bis zum Sieden. Nach 48 Stunden Rückfluss wurde das Lösemittel abgesaugt und die Katalysatorkugeln wurden bei 1,33 mmbar und 85 °C vier Stunden getrocknet. Der Jodidgehalt betrug 2,55 Gew%, das Lösemittel war jodidfrei.

Zu 3,2 g dieser Katalysatormasse gab man 70 mg (7,8 mg Rh) der Verbindung $[(C_2H_5O)_3SiCH_2CH_2P\text{Ø}_2]_2RhCOCl$, gelöst in 20 ml Xylol, unter Rühren hinzu. Die Suspension wurde bis zum Sieden erhitzt. Nach 36 h Rückfluss war die gelbe Lösung vollständig entfärbt. Das Lösemittel Xylol wurde unter vermindertem Druck abgesaugt und der gelbliche Katalysator in die Soxhlet-Apparatur überführt. Nach 12 h Soxhlet-Extraktion mit Benzol wurde der Katalysator bei 1,33 mbar und 85 °C 8 Stunden getrocknet und anschliessend mit Trimethylchlorsilan weiterbehandelt. Der Rhodium-Gehalt des so hergestellten Katalysators beträgt 0,24 Gew%.

Katalysator Nr. 7

$$[\overset{NaX}{Zeolith}\text{-})\text{-}O\text{-}\overset{OC_2H_5}{\underset{OC_2H_5}{Si}}\text{-}CH_2\text{-}CH_2P\text{Ø}_2]_2RhCOCl$$

Zu 25 g aktiviertem NaX-Zeolith von 2 mm Korndurchmesser und einer BET-Oberfläche von 800 $m^2/g$ gab man 1,07 g (112 mg Rh) der Verbindung $[(C_2H_5O)_3SiCH_2CH_2P\text{Ø}_2]_2RhCOCl$, gelöst in 100 ml Xylol, unter Rühren hinzu. Die Mischung wurde bis zum Sieden erhitzt. Nach 72 h Rückfluss war die gelbe Lösung vollständig entfärbt. Das Lösemittel wurde abgesaugt und der Katalysator in die Soxhlet-Apparatur überführt. Nach 12 h Soxhlet-Extraktion mit Benzol wurde der Katalysator bei 1,33 mbar und 85 °C acht Stunden getrocknet und anschliessend mit Trimethylchlorsilan weiterbehandelt. Der Rhodiumgehalt des so hergestellten Katalysators beträgt 0,26 Gew%.

Katalysator Nr. 8

$$[Al_2O_3\text{-})\text{-}O\text{-}\overset{OC_2H_5}{\underset{OC_2H_5}{Si}}\text{-}CH_2CH_2P\text{Ø}_2]_2RhCOCl$$

Der Katalysator wurde analog Katalysator Nr. 2 hergestellt. Der Rhodiumgehalt beträgt jedoch 0,6 Gew%.

Katalysator Nr. 9

$$[Al_2O_3\text{-})\text{-}O\text{-}\overset{OC_2H_5}{\underset{OC_2H_5}{Si}}CH_2CH_2P\text{Ø}_2]_2PdCl_2$$

Zu 3,1 g aktiviertem Aluminiumoxid (99% $Al_2O_3$) mit einem Korndurchmesser von 3 mm, einer inneren Oberfläche nach BET von 125 $m^2/g$ und einem Porenvolumen von 0,9 ml/g wurden 140 mg (15,8 mg Pd) der Verbindung $[(C_2H_5O)_3SiCH_2CH_2P\text{Ø}_2]_2PdCl_2$ (hergestellt aus $(C_2H_5O)_3SiCH_2CH_2P\text{Ø}_2$ und $Pd(CH_3CO_2)_2$ oder $(C_6H_5CN)_2PdCl_2$ oder $PdCl_2(C_8H_{12})$, vgl. J. Chem. Soc. (London) Chem. Com. 1977, S. 510), gelöst in einer Mischung aus je 10 ml Benzol und Dichlormethan, unter Rühren hinzugegeben und die Suspension zum Sieden erhitzt. Nach 56 h Rückfluss war die gelbe Lösung vollständig entfärbt. Das Lösemittelgemisch wurde abgesaugt und der gelbliche Katalysator in die Soxhlet-Apparatur überführt. Nach 12 h Soxhlet-Extraktion mit Benzol wurde der Katalysator bei 1,33 mbar und 85 °C acht Stunden getrocknet und anschliessend mit Trimethylchlorsilan weiterbehandelt. Die eingeengten Lösungen enthielten kein Palladium mehr. Der Palladiumgehalt des so hergestellten Katalysators beträgt 0,37 Gew%.

Katalysator Nr. 10

$$[Al_2O_3\text{-})\text{-}O\text{-}\overset{OC_2H_5}{\underset{OC_2H_5}{Si}}\text{-}CH_2\text{-}CH_2P\text{Ø}_2]_2Ru(CO)_2Cl_2$$

Zu 3,2 g aktiviertem Aluminiumoxid (99% $Al_2O_3$) mit einem Korndurchmesser von 3 mm, einer inneren Oberfläche nach BET von 125 $m^2/g$ und einem Porenvolumen von 0,9 ml/g wurden 140 mg (15,5 mg Ru) der Verbindung $[(C_2H_5)_3SiCH_2CH_2P\text{Ø}_2]_2Ru(CO)_2Cl_2$ (hergestellt aus $(C_2H_5O)_3SiCH_2CH_2P\text{Ø}_2$ und $(P\text{Ø}_3)_2RuCl_2(CO)_2$, vgl. Pittman, JACS 97 (1975), S. 1749), gelöst in einer Mischung von je 15 ml Dichlormethan und Benzol unter Rühren hinzugegeben. Die Suspension wurde bis zum Sieden erhitzt. Nach 72 h Rückfluss war die Lösung entfärbt. Nach Abtrennung des Lösemittelgemisches wurde der Katalysator in die Soxhlet-Apparatur überführt. Nach 12 h Soxhlet-Extraktion mit Benzol wurde der Katalysator bei 1,33 mbar und 85 °C acht Stunden getrocknet und anschliessend mit Trimethylchlorsilan behandelt. Die eingeengten Lösungen enthielten kein Ruthenium mehr. Der Rutheniumgehalt des so hergesellten Katalysators beträgt 0,48 Gew%.

Katalysator Nr. 11

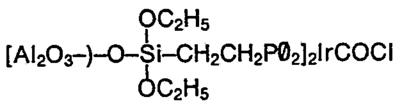

Zu 5,6 g aktiviertem Aluminiumoxid (99% $Al_2O_3$) mit einem Korndurchmesser von 3 mm, einer inneren Oberfläche nach BET von 125 $m^2/g$ und einem Porenvolumen von 0,9 ml/g wurden 125 mg (23,8 mg Ir) der Verbindung $[(C_2H_5O)_3SiCH_2CH_2PØ_2]_2IrCOCl$ (hergestellt aus $(C_2H_5O)_3SiCH_2CH_2PØ_2$ und $[(IrCl(C_8H_{12})]_2$ oder $Cl(CO)_2Irpyr$ oder $IrClCO(PØ_3)_2$, vgl. Pittmann, JACS 97 (1975) S. 4774), gelöst in 100 ml Toluol, hinzugegeben und bis zum Sieden erhitzt. Nach 100 h Rückfluss war die gelbe Lösung vollständig entfärbt. Nach Abtrennung des Lösemittels wurde der Katalysator in die Soxhlet-Apparatur überführt. Nach 12 h Soxhlet-Extraktion mit Benzol wurde der Katalysator bei 1,33 mbar und 85 °C acht Stunden getrocknet. Die eingeengten Lösemittel waren iridiumfrei. Der Iridiumgehalt des so hergestellten Katalysators beträgt 0,41 Gew%.

Katalysator Nr. 12

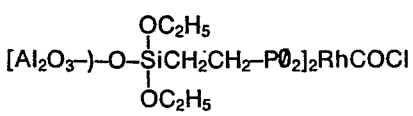

Der Katalysator wurde analog Katalysator Nr. 2 hergestellt. Der Rhodium-Gehalt beträgt 0,9 Gew%.

Katalysator Nr. 13

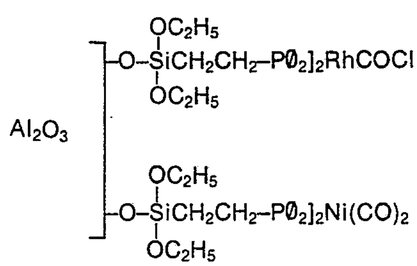

Zu 3,2 g aktiviertem Aluminiumoxid (99% $Al_2O_3$) mit einem Korndurchmesser von 3 mm, einer inneren Oberfläche nach BET von 125 $m^2/g$ und einem Porenvolumen von 0,9 ml/g gab man 100 mg (11,2 mg Rh) der Verbindung $[(C_2H_5O)_3SiCH_2CH_2PØ_2]_2RhCOCl$ und 100 mg (6,7 mg Ni) der Verbindung $[(C_2H_5O)_3SiCH_2CH_2PØ_2]_2Ni(CO)_2$ (hergestellt aus $(C_2H_5O)_3SiCH_2CH_2PØ_2$ und $[Rh(CO)_2Cl]_2$ bzw. $Ni(CO)_4$, vgl. A.K. Smith et al, J. mol. Catal. 2 (1977), S. 223), gelöst in 40 ml Xylol, unter Rühren hinzu. Die Suspension wurde bis zum Sieden erhitzt. Nach 72 h Rückfluss war die gelbe Lösung entfärbt. Nach Abtrennung des Lösemittels wurde der Katalysator in die Soxhlet-Apparatur überführt. Nach 12 h Soxhlet-Extraktion mit Benzol wurde der Katalysator bei 1,33 mbar und 85 °C acht Stunden getrocknet und anschliessend mit Trimethylchlorsilan behandelt. Die eingeengten Lösungen waren rhodium- und nickelfrei. Der Rhodiumgehalt des so hergestellten Katalysators beträgt 0,33 Gew%, Der Promotorgehalt an Nickel beträgt 0,19 Gew%.

Katalysator Nr. 14

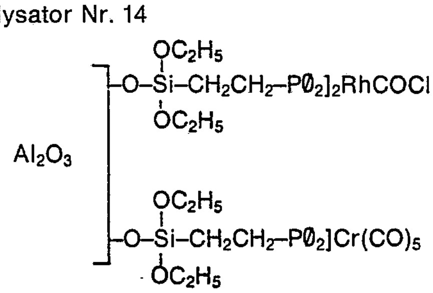

Zu 3,2 aktiviertem Aluminiumoxid (99% $Al_2O_3$) mit einem Korndurchmesser von 3 mm, einer inneren Oberfläche nach BET von 125 $m^2/g$ und einem Porenvolumen von 0,9 ml/g gab man 100 mg (11,2 mg Rh) der Verbindung $[(C_2H_5O)_3SiCH_2CH_2PØ_2]_2RhCOCl$ und 125 mg (11,4 mg Cr) der Verbindung $[(C_2H_5O)_3SiCH_2CH_2PØ_2]Cr(CO)_5$ (hergestellt aus $(C_2H_5)_3SiCH_2CH_2PØ_2$ und $[Rh(CO)_2Cl]_2$ bzw. $Cr(CO)_6$, vgl. JACS 81 (1959), S. 2273), gelöst in 40 ml Xylol, unter Rühren hinzu. Die Suspension wurde bis zum Sieden erhitzt. Nach 72 h Rückfluss war die gelbe Lösung vollständig entfärbt. Nach Abtrennung des Lösemittels wurde der Katalysator in die Soxhlet-Apparatur überführt. Nach 12 h Soxhlet-Extraktion mit Benzol wurde der Katalysator bei 1,33 mbar und 85 °C acht Stunden getrocknet und anschliessend mit Trimethylchlorsilan behandelt. Die eingeengten Lösemittel waren rhodium- und nickelfrei. Der Rhodiumgehalt des so hergestellten Katalysators beträgt 0,33 Gew%, der Promotorgehalt an Chrom beträgt 0,3 Gew%.

**Patentansprüche**

1. Verfahren zur Herstellung von Monocarbonsäureanhydriden der allgemeinen Formel $(RCO)_2O$ durch Umsetzung eines Carbonsäureesters oder Dialkylethers der allgemeinen Formeln RCOOR bzw. ROR, wobei R jeweils denselben Alkylrest mit 1–4 C-Atomen bedeutet, mit Kohlenmonoxid in der Gasphase in Gegenwart von Jod oder Brom oder deren Verbindungen als Reaktionspromotor sowie in Gegenwart eines Edelmetallverbindungen aus der Gruppe VIII des Periodensystems enthaltenden Trägerkatalysators bei Temperaturen von 130 bis 400 °C und Drücken von 1–150 bar, dadurch gekennzeichnet, dass im Trägerkatalysator eine Organosiliciumverbindung mit Alkoxy- oder Halogengruppe sowie mit Organostickstoff-, Organophosphor-, Organoarsen-, Organoschwefel-, Mercapto- oder Thioethergruppen als mehrfunktioneller Haftvermittler einerseits an das Trägermaterial und andererseits an die Edelmetallverbindung gebunden ist.

2. Verfahren nach Anspruch 1, dadurch gekennzeichnet, dass der Trägerkatalysator zusätzlich Nichtedelmetallverbindungen aus der 1. bis 3. Hauptgruppe oder der 4. bis 6. oder der 8. Nebengruppe des Periodensystems der Elemente als Promotoren enthält.

3. Verfahren nach Anspruch 1 oder 2, dadurch gekennzeichnet, dass im Trägerkatalysator die Organosiliciumverbindung als mehrfunktioneller Haftvermittler einerseits an das Trägermaterial und andererseits abwechselnd an die Edelmetallverbindung und an eine Nichtedelmetallverbindung aus der 6. oder 8. Nebengruppe des Periodensystems der Elemente gebunden ist.

4. Verfahren nach einem der Ansprüche 1 bis 3, dadurch gekennzeichnet, dass der mehrfunktionelle Haftvermittler eine Organosiliciumverbindung folgender allgemeiner Formeln ist:

I. $R^1_nX_{3-n}Si-(CR^3_2)_m-Y$ oder

II. $R^1_nX_{3-n}Si-(CR^3_2)_m-CHY_2$ oder

III. $[R^1_nX_{3-n}Si-(CR^3_2)_m]_2Z$,

wobei

X = $-Cl$, $-Br$ oder $-OR^2$;

Y = $-NR^4_2$, ein stickstoffhaltiger Arylrest, $-PR^4_2$, $-AsR^4_2$, $-SR^4$ oder $-SH$;

Z = $-NR^4-$, $-PR^4-$, $-AsR^4-$ oder $-S-$;

$R^1$ = $C_1$ bis $C_5$-Alkyl;

$R^2$ = $C_1$ bis $C_3$-Alkyl;

$R^3$ = $-H$, $C_1$ bis $C_5$-Alkyl oder $-C_6H_5$;

$R^4$ = $C_1$ bis $C_6$-Alkyl, $C_5$ bis $C_8$-Cycloalkyl oder $-C_6H_5$ oder $C_6H_5CH_2-$, die ggf. mit Halogen-, Methoxy-, Ethoxy- oder $C_1$ bis $C_3$-Alkyl substituiert sind;

n = 0 oder 1 oder 2;

m = 0 bis 8, vorzugsweise 1 bis 3.

5. Verfahren nach einem der Ansprüche 1–4, dadurch gekennzeichnet, dass der Trägerkatalysator ein anorganisches oxidisches Trägermaterial oder einen Aktivkohleträger enthält, deren restliche aktive Hydroxygruppen durch Veresterung oder Veretherung desaktiviert wurden.

6. Verfahren nach einem der Ansprüche 1–5, dadurch gekennzeichnet, dass der Trägerkatalysator zusammen 0,01 bis 50 Gew%, vorzugsweise 0,1 bis 20 Gew%, Edelmetallverbindung, Haftvermittler und ggf. Nichtedelmetallverbindung enthält.

7. Verfahren nach einem der Ansprüche 1–6, dadurch gekennzeichnet, dass der Trägerkatalysator in einer Korngrösse von 1 bis 20 mm eingesetzt wird.

## Claims

1. Process for making monocarboxylic anhydrides of the general formula $(RCO)_2O$ by reacting a carboxylic acid ester of the general formula RCOOR or dialkylether of the general formula ROR, in each of which formulae R stands for one and the same alkyl group having from 1 to 4 carbon atoms, with carbon monoxide in gas phase in the presence of iodine or bromine or their compounds as a reaction promoter and also in the presence of a carrier-supported catalyst containing a compound of a noble metal belonging to group VIII of the Periodic System, at temperatures of 130 to 400 °C and under pressures of 1 to 150 bars, characterized in that, in the carrier-supported catalyst, an organosilicon compound containing an alkoxy or halogen group and also an organonitrogen, organophosphorus, organoarsenic, organosulfur, mercapto or thioether group as a polyfunctional adhesion promoter is linked to the carrier material on the one hand, and to the noble metal compound on the other hand.

2. Process as claimed in claim 1, wherein the carrier-supported catalyst additionally contains as a promoter a compound of a non noble metal belonging to the 1st through 3rd principal groups or to the 4th through 6th or 8th subgroups of the Periodic System of the elements.

3. Process as claimed in claim 1 or 2, wherein the organosilicon compound as the polyfunctional adhesion promoter in the carrier-supported catalyst is linked to the carrier material on the one hand and alternately to the noble metal compound and to a non noble metal compound selected from the 6th or 8th subgroup of the Periodic System of the elements.

4. Process as claimed in any of claims 1–3, wherein the polyfunctional adhesion promoter is an organosilicon compound corresponding to one of the following general formulae:

I. $R^1_nX_{3-n}Si-(CR^3_2)_m-Y$ or

II. $R^1X_{3-n}Si-(CR^3_2)_m-CHY_2$ or

III. $[R^1_nX_{3-n}Si-(CR^3_2)_m]_2Z$

in which

X = $-Cl$, $-Br$ or $-OR^2$;

Y = $-NR^4_2$, a nitrogen-containing aryl group, $-PR^4_2$, $-AsR^4_2$, $-SR^4$ or $-SH$;

Z = $-NR^4-$, $-PR^4-$, $-AsR^4-$ or $-S-$;

$R^1$ = a $C_1$–$C_5$-alkyl;

$R^2$ = a $C_1$–$C_3$-alkyl;

$R^3$ = $-H$, a $C_1$–$C_5$-alkyl or $-C_6H_5$;

$R^4$ = a $C_1$–$C_6$-alkyl, a $C_5$–$C_8$-cycloalkyl or $-C_6H_5$ or $C_6H_5CH_2-$ which may be substituted with a halogen, methoxy group, ethoxy group or a $C_1$–$C_3$-alkyl;

n = 0 or 1 or 2;

m = 0 through 8, preferably 1–3.

5. Process as claimed in any of claims 1–4, wherein the carrier-supported catalyst contains inorganic oxidic carrier or an active carbon carrier the residual active hydroxy groups of which have been inactivated by esterification or etherification.

6. Process as claimed in any of claims 1–5, wherein the carrier-supported catalyst contains altogether 0.01 to 50 wgt %, preferably 0.1 to 20 wgt %, noble metal compound, adhesion promoter and non noble metal compound, if any.

7. Process as claimed in any of claims 1–6, wherein the carrier-supported catalyst is used in the form of particles having a size of 1 to 20 mm.

## Revendications

1. Procédé de préparation d'anhydrides monocarboxyliques de formule générale $(RCO)_2O$ par

réaction d'un ester carboxylique de formule générale RCOOR ou d'un éther dialkylique de formule générale ROR, dans lesquelles R représente chaque fois le même groupe alkyle en $C_1$–$C_4$, avec le monoxyde de carbone en phase gazeuse, en présence d'iode ou de brome ou de leurs composés comme promoteur de réaction, ainsi qu'en présence d'un catalyseur sur support contenant des composés d'un métal noble appartenant au groupe VIII de la Classification Périodique des Eléments, à des températures de 130–400 °C et sous des pressions de 1–150 bars, caractérisé en ce que, dans le catalyseur sur support, un composé organosilicié contenant des groupes alcoxyles ou halogénés ainsi que des groupes organoazotés, organophosphorés, organoarséniés, organosoufrés, des groupes mercapto ou thioéthers comme auxiliaire de fixation polyfonctionnel est lié au support d'une part et au composé de métal noble d'autre part.

2. Procédé selon la revendication 1, caractérisé en ce que le catalyseur sur support contient en outre comme promoteurs des composés métalliques de métaux non nobles appartenant aux groupes principaux 1–3 ou aux sous-groupes 4–6 ou 8 de la Classification Périodique des Eléments.

3. Procédé selon la revendication 1 ou 2, caractérisé en ce que le composé organosilicié servant d'auxiliaire de fixation polyfonctionnel est lié dans le catalyseur sur support au support, d'une part et, alternativement, au composé de métal noble et à un composé de métal non noble appartenant au sous-groupe 6 ou 8 de la Classification Périodique des Eléments, d'autre part.

4. Procédé selon l'une des revendications 1–3, caractérisé en ce que l'auxiliaire de fixation polyfonctionnel est un composé organosilicié répondant à l'une des formules générales suivantes:

I. $R^1_nX_{3-n}Si(CR^3_2)_m\text{-}Y$ ou

II. $R^1_nX_{3-n}Si(CR^3_2)_m\text{-}CHY_2$ ou

III. $[R^1_nX_{3-n}Si\text{-}(CR^3_2)_m]_2Z$

dans lesquelles

X = –Cl, –Br ou $-OR^2$;

Y = $-NR^4_2$, un groupe aryle azoté, $-PR^4_2$, $-AsR^4_2$, $-SR^4$ ou –SH;

Z = $-NR^4-$, $-PR^4-$, $-AsR^4-$ ou –S–;

$R^1$ = alkyle en $C_1$–$C_5$;

$R^2$ = alkyle en $C_1$–$C_3$;

$R^3$ = –H, alkyle en $C_1$–$C_5$ ou $-C_6H_5$;

$R^4$ = alkyle en $C_1$–$C_6$, cycloalkyle en $C_5$–$C_8$ ou $-C_6H_5$ ou $C_6H_5CH_2-$, éventuellement substitués par un halogène, un groupe méthoxy, éthoxy ou alkyle en $C_1$–$C_3$;

n = 0, 1 ou 2;

m = 0 à 8, de préférence 1 à 3.

5. Procédé selon l'une des revendications 1–4, caractérisé en ce que le catalyseur sur support contient un support d'oxyde inorganique ou un support de charbon actif dont les groupes hydroxyles actifs résiduaires ont été désactivés par estérification ou éthérification.

6. Procédé selon l'une des revendications 1–5, caractérisé en ce que le catalyseur sur support contient au total 0,01–50% en poids, de préférence 0,1–20% en poids, de composé de métal noble, d'auxiliaire de fixation et, éventuellement, d'autres composés de métaux non nobles.

7. Procédé selon l'une des revendications 1–6, caractérisé en ce que l'on utilise le catalyseur sur support à une granulométrie de 1–20 mm.